# EUROPEAN PATENT SPECIFICATION

(11) **EP 2 481 380 B1**
(45) Date of publication and mention of the grant of the patent: **10.01.2018**
(21) Application number: 12153358.2
(22) Date of filing: 31.01.2012
(51) Int. Cl.: A61F 2/95

(54) **Wire with compliant sheath**
Draht mit passender Ummantelung
Câble doté d'une gaine souple

(30) Priority: 01.02.2011 US 201113019229
(43) Date of publication of application: 01.08.2012
(73) Proprietor: Micrus Endovascular LLC, San Jose, California 95131 (US)
(72) Inventor: Ditter, Tom A., San Jose, California 95131 (US)
(74) Representative: Small, Gary James

(56) References cited:
- WO-A1-00/71058
- US-A1- 2002 013 599

## Description

### BACKGROUND OF THE INVENTION

The present application generally relates to systems for deploying and/or retrieving intravascular stents, and more particularly relates to an apparatus including an intravascular stent delivery wire having a compliant sheath mounted over the stent delivery wire for delivery and retrieval of an intravascular stent releasably placed over the compliant sheath.

Intravascular stents are generally tubular and are actively or passively expanded radially in the vasculature of a patient. Such stent can be mounted over an expandable member or balloon of a balloon angioplasty catheter, for deployment of the stents by expansion of the balloon at the treatment site of the vasculature, such as at a stenosis or an aneurysm. Self-expanding stents can expand from a compressed delivery position to a larger diameter without the assistance of an expandable member or balloon.

Intravascular stent delivery systems for placement of an intravascular self-expanding stent at a treatment site in the vasculature typically have included a catheter that can be threaded through the vasculature with the self-expanding stent axially placed over a distal portion of the catheter. To position an intravascular stent at the treatment site of the vasculature, such as at a stenosis or an aneurysm, a guiding catheter typically is introduced into the vascular system of a patient, and advanced within the vasculature until the distal tip of the guiding catheter is adjacent to the treatment site. A guidewire is typically then advanced through the guiding catheter to the desired location, and then a dilatation or delivery catheter having a stent positioned on the catheter is advanced into the patient's vasculature over the guidewire, until the stent is properly positioned, after which the stent can be deployed at the treatment site.

One common technique for maintaining a self-expanding stent in a low profile configuration involves placement of a sheath or a sleeve over some or all of the stent, typically either to retain the stent in a compressed configuration around the catheter, to prevent body fluids from reaching the stent, or to protect the vasculature from the stent. Usually such a sheath or sleeve is retracted or released from the stent to allow the stent to achieve an expanded configuration.

A guidewire loaded stent is also known in which a radially expandable stent carried on a guidewire is covered in part by a retractable sheath at or near a distal end of the guidewire. When the stent is adjacent to a treatment site, the sheath is retracted to expose the stent to allow the stent to expand. Document WO00/71058 discloses an apparatus comprising an intravascular delivery wire, over which a ring of compliant material is mounted.

It would be desirable to provide an intravascular stent delivery system including an intravascular stent delivery wire having a compliant sheath mounted over the stent delivery wire for delivery and retrieval of an intravascular stent releasably placed over the compliant sheath, in order to provide a reduced diameter profile to permit delivery of an intravascular stent to smaller diameter and more delicate vessels of the vasculature, such as the neurovasculature. The present invention meets these and other needs.

### SUMMARY OF THE INVENTION

Insofar as the terms "invention" and/or "embodiment" are used in the following, and/or features are presented as being optional, this should be interpreted in such a way that the only protection sought is that of the invention as claimed. Briefly and in general terms, the present invention provides for an apparatus for deploying and/or retrieving intravascular stents, including an intravascular stent delivery wire having a compliant sheath mounted over the stent delivery wire for delivery and retrieval of an intravascular stent releasably placed over the compliant sheath.

The present invention accordingly provides for an apparatus for deployment and retrieval of a self-expanding intravascular stent or a self-expanding intravascular stent-like device, including an intravascular delivery wire, and one or more bands or rings of compliant material fixedly mounted over at least a portion of the intravascular delivery wire. In a presently preferred aspect, the compliant material can be an elastomeric or polymeric material, such as silicone or polyurethane, or a tunably compliant polymeric material that can be caused to undergo a change in stiffness or compliance when subjected to temperature changes, or electricity, such as an electroactive polymer (EAP), for example. In a presently preferred aspect, the one or more bands or rings of compliant material are fixedly mounted over a distal portion of the intravascular delivery wire. In another presently preferred aspect, the one or more bands or rings of compliant material include a plurality of rings of compliant material.

The intravascular delivery wire is a stepped intravascular delivery wire, wherein the distal portion of the intravascular delivery wire is a stepped distal portion having a smaller diameter than the proximal portion of the intravascular delivery wire, forming a hard stop proximal to the one or more bands or rings of compliant material to restrict or prevent movement of a self-expanding intravascular stent releasably mounted over the one or more bands or rings of compliant material proximally over the intravascular delivery wire.

In another presently preferred aspect, a distal band or ring of compliant or non-compliant material having a diameter larger than a diameter of the intravascular delivery wire is fixedly mounted to the intravascular delivery wire distal to the one or more rings of compliant material, and a proximal band or ring of compliant or non-compliant material having a diameter larger than a diameter of the intravascular delivery wire is fixedly mounted to the intravascular delivery wire proximal to the one or more rings of compliant material. In a presently preferred aspect, the distal and proximal bands or rings of compliant or non-compliant material form hard stops configured to restrict movement of the self-expanding intravascular stent or a self-expanding intravascular stent-like device until the self-expanding intravascular stent or self-expanding intravascular stent-like device has achieved an expanded configuration. In another presently preferred aspect, the distal and proximal bands are radiopaque marker bands.

In another presently preferred aspect, a self-expanding intravascular stent is releasably retained on the one or more bands or rings of compliant material, such as by pressing or crimping the self-expanding intravascular stent on the one or more bands or rings of compliant material, to releasably mount the self-expanding intravascular stent over the one or more bands or rings of compliant material on the intravascular delivery wire.

These and other features and advantages of the present invention will become more apparent from the following detailed description of the preferred embodiments in conjunction with the accompanying drawings, which illustrate, by way of example, the operation of the invention.

In accordance with the present invention, there is provided an apparatus for deployment and retrieval of a self-expanding intravascular stent, the apparatus comprising an intravascular delivery wire having a distal portion and a proximal portion; and at least one ring of compliant material fixedly mounted over at least a portion of the intravascular delivery wire, said at least one ring of compliant material being configured to releasably mount a self-expanding intravascular stent on said intravascular delivery wire.

### BRIEF DESCRIPTION OF THE DRAWINGS

Figure 1 is a schematic diagram of an end view of a first embodiment of the apparatus for deploying and/or retrieving intravascular stents, according to the invention.
Fig. 2 is a sectional view of the apparatus for deploying and/or retrieving intravascular stents, taken along line 2-2 of Fig. 1.
Fig. 3 is a schematic diagram illustrating a stent mounted over the apparatus for deploying and/or retrieving intravascular stents of Fig. 1.
Fig. 4 is a schematic diagram illustrating a stent mounted over a second embodiment of the apparatus for deploying and/or retrieving intravascular stents, according to the invention.
Fig. 5 is a schematic diagram illustrating a stent mounted over a third embodiment of the apparatus for deploying and/or retrieving intravascular stents, according to the invention.
Fig. 6 is a schematic diagram illustrating a stent mounted over a fourth embodiment of the apparatus for deploying and/or retrieving intravascular stents, according to the invention.

### DETAILED DESCRIPTION OF THE PREFERRED EMBODIMENTS

Referring to the drawings, which are provided by way of example, and not by way of limitation, the present invention provides for a system and apparatus for deploying and/or retrieval of a self-expanding intravascular stent or a intravascular stent-like device.

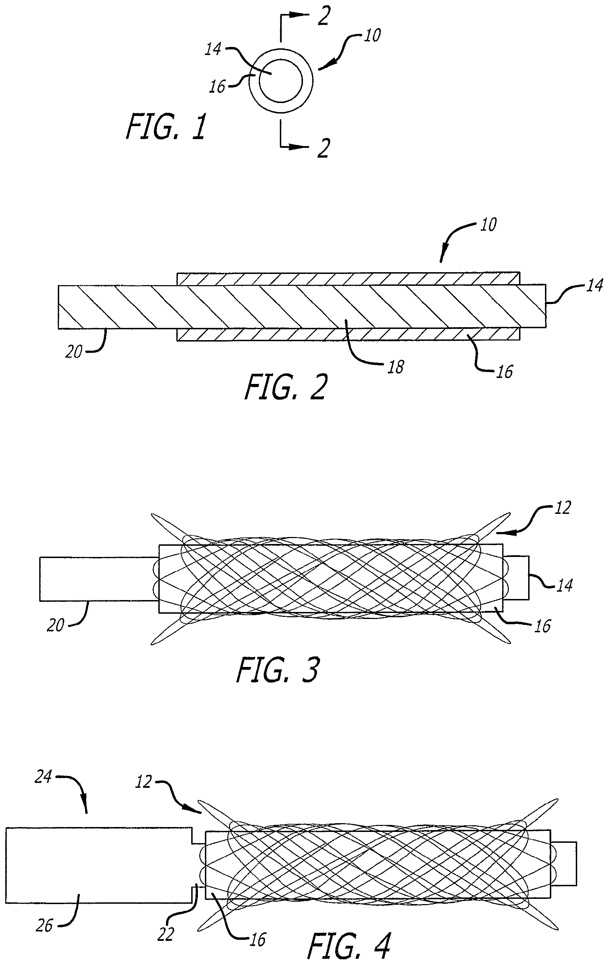

Accordingly, an apparatus 10 for deploying and/or retrieval of a self-expanding intravascular stent 12 or a self-expanding intravascular stent-like device includes an intravascular delivery wire 14, over which one or more bands, rings, or sheaths of compliant material 16 is overlaid. The compliant material is defined herein as a compliant material that undergoes elastic deformation when subjected to an applied force. The compliant material can be an elastomeric or polymeric material, such as silicone or polyurethane, for example, although other similar materials that undergo elastic deformation when a stent or stent-like device is pressed or crimped on the compliant material may be suitable. Alternatively, the compliant material can be a tunably compliant polymeric material that can be caused to undergo a change in stiffness or compliance when subjected to temperature changes, such as undergoing a softening when heated, or that can be caused to undergo a change in stiffness or compliance when subjected to controlled application of electrical voltage or current, such as an electroactive polymer (EAP), for example. As is illustrated in Figs. 1-3, at least one band, ring, or sheath of compliant material is fixedly mounted over at least a portion of an intravascular delivery wire, such as a distal portion 18 of the intravascular delivery wire, which can have the same diameter as a proximal portion 20 of the intravascular delivery wire.

As is illustrated in Fig. 4, in a second embodiment, the compliant material is overlaid onto a distal stepped portion 22 of a stepped intravascular delivery wire 24, which is typically ground down or otherwise formed such that the distal stepped portion has a smaller diameter than a proximal portion 26 of the intravascular delivery wire. The distal stepped portion of the stepped intravascular delivery wire forms a hard stop proximal to the stent to block the self-expanding intravascular stent or a self-expanding intravascular stent-like device from advancing proximally over the intravascular delivery wire when the self-expanding intravascular stent-like device achieves an expanded configuration.

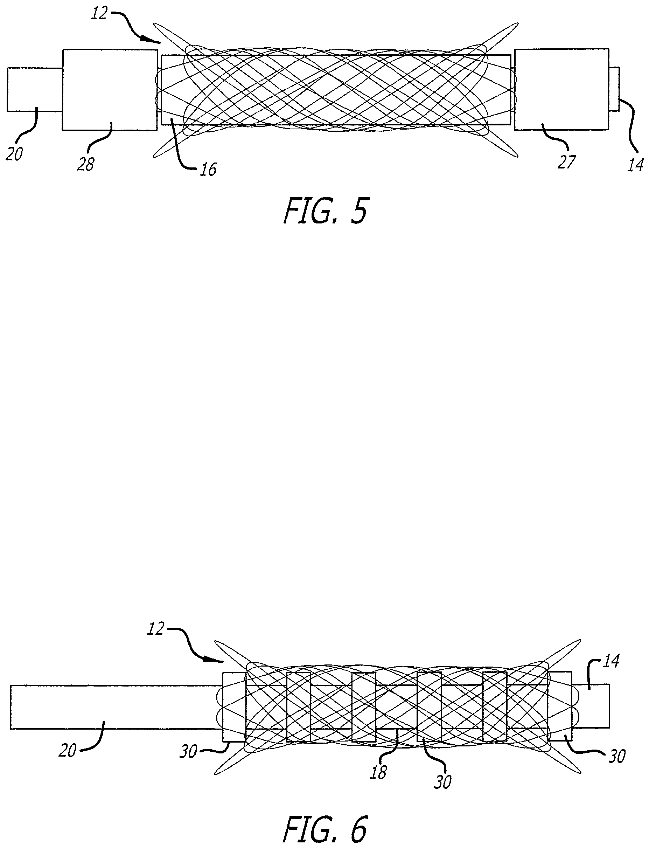

In a third embodiment, illustrated in Fig. 5, a distal band or ring 27 of compliant or non-compliant material is fixedly mounted to the intravascular delivery wire distal to the one or more bands or rings of compliant material, and a proximal band or ring 28 of compliant or non-compliant material is fixedly mounted to the intravascular delivery wire proximal to the one or more bands or rings of compliant material. Each of the distal and proximal bands or rings of compliant or non-compliant material preferably has a diameter larger than a diameter of the intravascular delivery wire, and can be formed in or fixedly mounted on the delivery wire distal and proximal to the one or more bands or rings of compliant material, so that the compliant material is situated in between the distal and proximal bands or rings. The distal and proximal bands or rings can be formed of a radiopaque material, such as a radiopaque metal, for example, to form radiopaque marker bands, and can be formed to have a diameter larger than the diameter of the one or more bands or rings of compliant material, to also act as hard stops to restrict movement of a self-expanding intravascular stent or a self-expanding intravascular stent-like device releasably mounted over the one or more bands or rings of compliant material, until the self-expanding intravascular stent or self-expanding intravascular stent-like device has achieved an expanded configuration.

In a fourth embodiment, illustrated in Fig. 6, the compliant material may be formed by a plurality or series of bands or rings of compliant material 30, such as several spaced apart bands or rings of compliant material, rather than a continuous sheath.

In each of the foregoing embodiments, a self-expanding intravascular stent or self-expanding intravascular stent-like device can be pressed or crimped onto the band, ring or sheath of compliant material or series of bands or rings of compliant material, to mount the self-expanding intravascular stent or intravascular stent-like device over the compliant material on the intravascular delivery wire. Friction between the compliant material and the intravascular stent allows the intravascular stent to be releasably retained on the compliant material for purposes of deployment and retrieval of the intravascular stent. If the compliant material is sufficiently compliant, the stent can be embedded into the layer of compliant material, which will further enhance deployment/retrievability of the intravascular stent.

It will be apparent from the foregoing that while particular forms of the invention have been illustrated and described, various modifications can be made without departing from the scope of the invention. Accordingly, it is not intended that the invention be limited, except as by the appended claims.

## Claims

1. An apparatus (10) for deployment and retrieval of a self-expanding intravascular stent (12), the apparatus comprising:
an intravascular delivery wire (24) having a distal portion and a proximal portion; and
at least one ring of compliant material (16) fixedly mounted over at least a portion of the intravascular delivery wire (24), said at least one ring of compliant material (16) being configured to releasably mount a self-expanding intravascular stent (12) on said intravascular delivery wire (24),
**characterized in that** said intravascular delivery wire (24) comprises a stepped intravascular delivery wire, wherein said distal portion of the intravascular delivery wire is a stepped distal portion (22) having a smaller diameter than said proximal portion (26) of the intravascular delivery wire (24),
wherein said stepped portion (22) of said stepped intravascular delivery wire (24) forms a hard stop proximal to said at least one ring of compliant material (16) operative to restrict movement of a self-expanding intravascular stent (12) releasably mounted over said at least one ring of compliant material (16) from advancing proximally over the intravascular delivery wire (24).

2. The apparatus of Claim 1, further comprising a distal band having a diameter larger than a diameter of the intravascular delivery wire fixedly mounted to said intravascular delivery wire distal to said at least one ring of compliant material, and a proximal band having a diameter larger than a diameter of the intravascular delivery wire fixedly mounted to said intravascular delivery wire proximal to said at least one ring of compliant material.

3. The apparatus of Claim 2, wherein said distal and proximal bands comprise radiopaque marker bands.

4. The apparatus of Claim 2 or 3, wherein said distal band and said proximal band form hard stops configured to restrict movement of the self-expanding intravascular stent or a self-expanding intravascular stent-like device until the self-expanding intravascular stent or self-expanding intravascular stent-like device has achieved an expanded configuration

5. The apparatus of any preceding claim, wherein said at least one ring of compliant material is fixedly mounted over said distal portion of the intravascular delivery wire.

6. The apparatus of any preceding claim, wherein said distal portion of the intravascular delivery wire has the same diameter as the proximal portion of the intravascular delivery wire.

7. The apparatus of any preceding claim, wherein said at least one ring of compliant material comprises a plurality of rings of compliant material.

8. The apparatus of any preceding claim, further comprising a self-expanding intravascular stent releasably retained on said at least one ring of compliant material to mount the self-expanding intravascular stent over said at least one ring of compliant material on the intravascular delivery wire.

9. The apparatus of claim 8, wherein said self-expanding intravascular stent is crimped onto said at least one ring of compliant material.

10. The apparatus of any preceding claim, wherein said compliant material comprises silicone.

11. The apparatus of any of claims 1 to 10, wherein said compliant material comprises polyurethane.

## Patentansprüche

1. Vorrichtung (10) zum Ablegen und Rückholen eines selbstexpandierenden intravaskulären Stents (12), wobei die Vorrichtung Folgendes umfasst:
einen intravaskulären Zuführdraht (24) mit einem distalen Abschnitt und einem proximalen Abschnitt und
mindestens einen Ring aus nachgiebigem Material (16), der fest über mindestens einem Abschnitt des intravaskulären Zuführdrahts (24) montiert ist, wobei der mindestens eine Ring aus nachgiebigem Material (16) dazu konfiguriert ist, einen selbstexpandierenden intravaskulären Stent (12) freigebbar an dem intravaskulären Zuführdraht (24) zu montieren,
**dadurch gekennzeichnet, dass** der intravaskuläre Zuführdraht (24) einen gestuften intravaskulären Zuführdraht umfasst, wobei der distale Abschnitt des intravaskulären Zuführdrahts ein gestufter distaler Abschnitt (22) mit einem Durchmesser ist, der kleiner als der des proximalen Abschnitts (26) des intravaskulären Zuführdrahts (24) ist,
wobei der gestufte Abschnitt (22) des gestuften intravaskulären Zuführdrahts (24) proximal zu dem mindestens einen Ring aus nachgiebigem Material (16) einen harten Anschlag bildet, der dazu dient, die Bewegung eines selbstexpandierenden intravaskulären Stents (12), der freigebbar über dem mindestens einen Ring aus nachgiebigem Material (16) montiert ist, dahingehend einzuschränken, dass er nicht proximal über den intravaskulären Zuführdraht (24) vorgeschoben wird.

2. Vorrichtung nach Anspruch 1, ferner umfassend ein distales Band mit einem Durchmesser, der größer als ein Durchmesser des intravaskulären Zuführdrahts ist, wobei das distale Band distal zu dem mindestens einen Ring aus nachgiebigem Material fest an dem intravaskulären Zuführdraht montiert ist, und ein proximales Band mit einem Durchmesser, der größer als ein Durchmesser des intravaskulären Zuführdrahts ist, wobei das proximale Band proximal zu dem mindestens einen Ring aus nachgiebigem Material fest an dem intravaskulären Zuführdraht montiert ist.

3. Vorrichtung nach Anspruch 2, wobei das distale und das proximale Band röntgendichte Markierungsbänder umfassen.

4. Vorrichtung nach Anspruch 2 oder 3, wobei das distale Band und das proximale Band harte Anschläge bilden, die dazu konfiguriert sind, die Bewegung des selbstexpandierenden intravaskulären Stents oder einer selbstexpandierenden intravaskulären stentartigen Einrichtung so lange einzuschränken, bis der selbstexpandierende intravaskuläre Stent oder die selbstexpandierende intravaskuläre stentartige Einrichtung eine expandierte Konfiguration erreicht hat.

5. Vorrichtung nach einem der vorhergehenden Ansprüche, wobei der mindestens eine Ring aus nachgiebigem Material fest über dem distalen Abschnitt des intravaskulären Zuführdrahts montiert ist.

6. Vorrichtung nach einem der vorhergehenden Ansprüche, wobei der distale Abschnitt des intravaskulären Zuführdrahts denselben Durchmesser wie der proximale Abschnitt des intravaskulären Zuführdrahts hat.

7. Vorrichtung nach einem der vorhergehenden Ansprüche, wobei der mindestens eine Ring aus nachgiebigem Material eine Vielzahl von Ringen aus nachgiebigem Material umfasst.

8. Vorrichtung nach einem der vorhergehenden Ansprüche, ferner umfassend einen selbstexpandierenden intravaskulären Stent, der freigebbar an dem mindestens einen Ring aus nachgiebigem Material gehalten ist, um den selbstexpandierenden intravaskulären Stent über dem mindestens einen Ring aus nachgiebigem Material an dem intravaskulären Zuführdraht zu montieren.

9. Vorrichtung nach Anspruch 8, wobei der selbstexpandierende intravaskuläre Stent auf den mindestens einen Ring aus nachgiebigem Material gecrimpt ist.

10. Vorrichtung nach einem der vorhergehenden Ansprüche, wobei das nachgiebige Material Silicon umfasst.

11. Vorrichtung nach einem der Ansprüche 1 bis 10, wobei das nachgiebige Material Polyurethan umfasst.

## Revendications

1. Appareil (10) pour la mise en place et la récupération d'un stent intravasculaire auto-expansible (12), l'appareil comprenant :
un fil de pose intravasculaire (24) comportant une partie distale et une partie proximale ; et
au moins un manchon de matériau souple (16) installé de manière fixe sur au moins une partie du fil de pose intravasculaire (24), ledit ou lesdits manchons de matériau souple (16) étant configurés pour installer de manière libérable un stent intravasculaire auto-expansible (12) sur ledit fil de pose intravasculaire (24),
**caractérisé en ce que** ledit fil de pose intravasculaire (24) comprend un fil de pose intravasculaire crénelé, ladite partie distale du fil de pose intravasculaire étant une partie distale en créneau (22) présentant un diamètre inférieur à celui de ladite partie proximale (26) du fil de pose intravasculaire (24),
ladite partie en créneau (22) dudit fil de pose intravasculaire (24) crénelé formant une butée fixe proximale vis-à-vis dudit ou desdits manchons de matériau souple (16) pouvant agir pour empêcher un stent intravasculaire auto-expansible (12) installé de manière libérable sur ledit ou lesdits manchons de matériau souple (16) d'avancer dans la direction proximale sur le fil de pose intravasculaire (24).

2. Appareil selon la revendication 1, comprenant en outre une bague distale présentant un diamètre supérieur à un diamètre du fil de pose intravasculaire installée de manière fixe sur ledit fil de pose intravasculaire en position distale vis-à-vis dudit ou desdits manchons de matériau souple, et une bague proximale présentant un diamètre supérieur à un diamètre du fil de pose intravasculaire installée de manière fixe sur ledit fil de pose intravasculaire en position proximale vis-à-vis dudit ou desdits manchons de matériau souple.

3. Appareil selon la revendication 2, dans lequel lesdites bagues distale et proximale comprennent des bagues de marquage radio-opaques.

4. Appareil selon la revendication 2 ou 3, dans lequel ladite bague distale et ladite bague proximale forment des butées fixes configurées pour limiter un déplacement du stent intravasculaire auto-expansible ou d'un dispositif semblable à un stent intravasculaire auto-expansible jusqu'à ce que le stent intravasculaire auto-expansible ou le dispositif semblable à un stent intravasculaire auto-expansible ait adopté une configuration déployée.

5. Appareil selon l'une quelconque des revendications précédentes, dans lequel ledit ou lesdits manchons de matériau souple sont installés de manière fixe sur ladite partie distale du fil de pose intravasculaire.

6. Appareil selon l'une quelconque des revendications précédentes, dans lequel ladite partie distale du fil de pose intravasculaire présente le même diamètre que la partie proximale du fil de pose intravasculaire.

7. Appareil selon l'une quelconque des revendications précédentes, dans lequel ledit ou lesdits manchons de matériau souple comprennent une pluralité de manchons de matériau souple.

8. Appareil selon l'une quelconque des revendications précédentes, comprenant en outre un stent intravasculaire auto-expansible retenu de manière libérable sur ledit ou lesdits manchons de matériau souple pour installer le stent intravasculaire auto-expansible sur ledit ou lesdits manchons de matériau souple sur le fil de pose intravasculaire.

9. Appareil selon la revendication 8, dans lequel ledit stent intravasculaire auto-expansible est serti sur ledit ou lesdits manchons de matériau souple.

10. Appareil selon l'une quelconque des revendications précédentes, dans lequel ledit matériau souple comprend du silicone.

11. Appareil selon l'une quelconque des revendications 1 à 10, dans lequel ledit matériau souple comprend du polyuréthane.
